(19) Europäisches Patentamt
European Patent Office
Office européen des brevets

(11) Publication number: **0 000 951**
**B1**

(12) # EUROPEAN PATENT SPECIFICATION

(45) Date of publication of patent specification: **23.05.84**

(51) Int. Cl.$^3$: **C 07 D 233/58,** A 61 K 31/415

(21) Application number: **78100754.7**

(22) Date of filing: **25.08.78**

(54) **Pharmaceutical Formulations comprising 1-substituted imidazoles, 1-substituted imidazoles and 1-substituted imidazoles for use in the treatment or prophylaxis of thrombo-embolic disorders.**

(30) Priority: **26.08.77 GB 3591277**
**26.08.77 GB 3591377**
**01.02.78 GB 398378**
**01.02.78 GB 398478**
**08.08.78 GB 3252678**
**08.08.78 GB 3253678**
**22.08.78 GB 3410678**

(43) Date of publication of application:
**07.03.79 Bulletin 79/05**

(45) Publication of the grant of the patent:
**23.05.84 Bulletin 84/21**

(84) Designated Contracting States:
**BE CH DE FR GB LU NL SE**

(73) Proprietor: **THE WELLCOME FOUNDATION LIMITED**
**183-193 Euston Road**
**London NW1 2BP (GB)**

(72) Inventor: **Thorogood, Peter Brian**
**114 Venner Road Sydenham**
**London, S.E. 26 (GB)**

(74) Representative: **Berg, Wilhelm, Dr. et al**
**Dr. Berg, Dipl.-Ing. Stapf, Dipl.-Ing. Schwabe, Dr. Dr. Sandmair Patentanwälte Postfach 860245**
**D-8000 München 86 (DE)**

(56) References cited:
**DE-A-2 533 211**
**DE-B-1 213 413**
**FR-A-1 603 783**
**GB-A-1 364 312**

**The file contains technical information submitted after the application was filed and not included in this specification**

(56) References cited:
**BIOCHEMICAL PHARMACOLOGY, 23, 2377-2386 (1974)**

**BIOCHEMICAL AND BIOPHYSICAL RESEARCH COMMUNICATIONS, VOL. 80, No. 1, 1978, pp. 236-242**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 000 951 B1

**Description**

The present invention relates to imidazole derivatives and salts thereof, to their synthesis and intermediates therefor, to pharmaceutical formulations containing such compounds and to the use of these componds in medicine.

Thromboxane $A_2$ ($TXA_2$), a potent stimulator of blood platelet aggregation, is produced, in platelets, from the prostaglandin endoperoxides $PGG_2$ and $PGH_2$. Prostacyclin ($PGI_2$), which has potent anti-aggregatory activity, is also produced (in blood vessel walls) from $PGG_2$ and $PGH_2$ and it has been suggested that a balance between the production of $TXA_2$ and $PGI_2$ is the controlling factor in thrombus formation. It would, in consequence, be desirable in the treatment and prophylaxis of thrombo-embolic disorders to be able to selectively inhibit $TXA_2$ synthetase, thereby favouring the production of the anti-aggregatory agent $PGI_2$.

Imidazole and 1-methylimidazole are known to provide some degree of inhibition of the enzymic conversion of the endoperoxides ($PGG_2$ and $PGH_2$) to thromboxane $A_2$ by platelet microsomes (Moncada *et al.*, Prostaglandins, *13*/4, 611—618, 1977). Certain 1-($C_{10-19}$)hydrocarbyl-imidazoles have been described as being capable of lowering serum cholesterol levels (U.K. Patent No. 1 364 312; Biochem. Pharmacol. *24*, 1902—1903, 1975).

Also 1-cyclohexylmethyl-imidazole is disclosed in Wiss. Z.Univ. Halle, Math-Nat. XI/5, pages 603—612, although no physiological activity is ascribed therein to the compound.

German Auslegeschrift 1213413 describes a process for the preparation of a broad class of 1-alkenyl and 1-alkylimidazoles optionally substituted by alkyl groups in the 2,3- and/or 5-positions. The resulting imidazoles are described in the specification as being useful as intermediates for the preparation of pest control and textile treatment agents.

The inhibitory potency of several derivatives of imidazole and 1-substituted imidazoles, such as 1-methylimidazole on thromboxane synthetase is known from Biochemical and Biophysical Research Communications, Vol. 80, No. 1 (1978), 236—242. The authors of this article report that the inhibitory potency of 1-substituted imidazoles is increased with increasing chain length, and state that 1-nonyl-imidazole and 1-(2-isopropylphenyl)imidazole were found to possess the highest potency. The best activities are obtained with compounds wherein the 1-substituent on the imidazole ring is a straight chain alkyl group. The reference gives no information as to the 1-substituted imidazoles carrying cycloalkyl or cycloalkenyl groups of 4 to 9 carbon atoms or alkyl radicals of from 4 to 7 carbon atoms or alkenyl or alkynyl radicals of from 4 to 9 carbon atoms.

We have now discovered that $TXA_2$ synthetase may be inhibited by 1-substituted imidazoles of formula (I) and pharmaceutically acceptable acid addition salts thereof. The compounds of formula (I) and their salts are hereinafter referred to as the "active compounds".

The compounds of formula (I) are:—

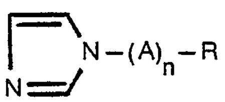

(I)

in which A is a straight or branched, saturated or unsaturated acyclic hydrocarbon radical of from 1 to 3 carbon atoms, n is 0 or 1, and R is a cycloalkyl or cycloalkenyl radical of from 4 to 9, preferably from 5 to 8, carbon atoms and optionally substituted by one, two, three or more alkyl radicals each containing from 1 to 4 carbon atoms, or, when n is 1, A and R together form a straight-chain alkyl radical of from 4 to 7 carbon atoms or an alkenyl or alkynyl group of from 4 to 9 carbon atoms.

A novel class of compounds within the scope of formula (I) are those of formula (Ia):

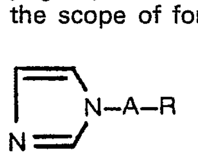

(Ia)

in which A is a straight or branched, saturated or unsaturated acyclic hydrocarbon radical of from 1 to 3 carbon atoms and R is a cycloalkyl or cycloalkenyl radical of from 4 to 9, preferably from 5 to 8, carbon atoms and optionally substituted by one or more alkyl radical each containing from 1 to 4 carbon atoms, with the proviso that when A is a methylene radical, R is not unsubstituted cyclohexyl. These compounds are described and claimed in our copending Application 78100753.9 (specification 0000951A1).

The following classes of compounds within the scope of formula (I) are also new and constitute features of the present invention:—

a) compounds of formula (I) wherein $(A)_n$ and R together form an alkenyl radical of from 5 to 9 carbon atoms.

b) compounds of formula (I) wherein n is 0 and R is a cycloalkyl or cycloalkenyl group of from 4 to 9 carbon atoms, optionally substituted by one or more alkyl radicals each containing 1 to 4 carbon

atoms, with the proviso that R is not unsubstituted cyclohexyl, and is also, preferably, not substituted cyclohexyl.

In formula (I) and (Ia) examples of cycloalkyl radicals are cyclobutyl, cyclopentyl, cyclohexyl, cycloheptyl and cyclooctyl; cycloalkenyl radicals include cyclohex-3-enyl, cyclopentyl, and 1,4-cyclohexadienyl; alkenyl radicals include pent-2-enyl and pent-4-enyl.

A valuable class of compounds of formula (I) are those in which n is 1, R is cyclohexyl, cyclohexyl, cycloheptyl, cyclooctyl, or cycloalkenyl of 6 to 8 carbon atoms, and A is $—CH_2$ or $—(CH_2)_2—$. Compounds of formula (I) or (Ia) may also be used pharmaceutically acceptable acid addition salts. Especially preferred compounds include:

1-Cyclooctylmethylimidazole
1-Cyclohex-3-enylmethylimidazole
1-Cyclohexylethylimidazole
1-Cycloheptylmethylimidazole

and pharmaceutically acceptable acid additions salts thereof

Other preferred compounds include:

1-Cyclopentylmethylimidazole
1-Cyclooctylvinylimidazole
1-(1-Cyclooctylethyl)imidazole
1-(2-Cyclooctylethyl)imidazole
1-(3-Cyclooctylpropyl)imidazole
1-(Cyclohept-2-enylmethyl)imidazole
1-Cyclononylmethylimidazole
1-(4-Methylcyclohexylmethyl)imidazole
1-(Cyclobutylmethyl)imidazole
1-Cycloheptylimidazole
1-Cyclopentylimidazole
1-Cyclohexylmethylimidazole
1-*n*-Butylimidazole
1-*n*-Pentylimidazole
1-*n*-Hexylimidazole
1-Pent-2-enylimidazole
1-Pent-4-enylimidazole
1-(3-Methylbutyl)imidazole

and pharmaceutically acceptable acid addition salts thereof.

In contrast to imidazole and 1-methylimidazole the compounds of formula (I) are more potent inhibitors of $TXA_2$ synthetase. Many of the compounds (for example in (I) R is cycloalkyl or cycloalkenyl, n is 1, and A is $—CH_2—$ or $—(CH_2)_2—$ are also more selective in their action in not inhibiting other prostaglandin-generating enzymes such as cyclo-oxygenase. The compounds of formula (I) also do not produce the side-effects found with imidazole upon *in vivo* administration. The compounds of formula (I) are further capable of inhibiting platelet aggregation *in vivo* and also are capable of disaggregating platelet clumps. The compounds 1-cyclooctylmethylimidazole, 1-cyclohex-3-enylmethylimidazole and 1-cyclohexylethylimidazole and their salts especially displaying these properties.

Imidazoles of formula (I) and acid addition salts thereof may be made by any method known in the art for the synthesis of compounds of analogous structure. In general these methods comprise linking the imidazole ring to the remainder of the molecule; converting a precursor molecule by elimination of a functional group from the imidazole ring; and formation of the desired compound from a corresponding imidazoline, pyrazole or unsaturated analogue.

A most convenient method of synthesis involves the reaction of imidazole (formula II) or a salt thereof with an alkylating agent of formula (III):

(II) 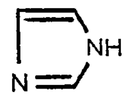 Z—A—R (III)

wherein R and A are as defined in formula (I) and Z is a leaving group. This reacion is well established in the literature, and the leaving group may be chosen from a variety of substituents but especially halo, preferably chloro or bromo, or from p-toluenesulphonyloxy but other arylsulphonyloxy, alkanesulphonyloxy or aralkylsulphonyloxy radicals may be used. The reaction is preferably performed in the presence of an acid acceptor, for example an alkali metal alkoxide such as sodium methoxide or potassium tertiary butoxide in the presence of a corresponding alkanol. The leaving group Z may itself be formed *in situ* from the corresponding alkanol (Z = OH) by reaction with a hydrohalogenic acid (e.g.

hydrochloric acid or a Lewis acid such as aluminium chloride: see Japanese Patent Kokai No. 131577/77) and the resulting agent of formula (III) reacted directly with imidazole without prior isolation. Alternatively an alkanol (Z= OH) or a derivative thereof (e.g. Z = R—A—O—) may be reacted directly with imidazole (II) by heating in the presence of a dehydrating agent such as phosphate acid, or a phosphate (see Japanese Patent Publication No. 5 1105 060), sulphuric acid or sulphates (see Japanese Patent Publication No. 5 1105 061).

Among precursor molecules which may be converted to a compound of formula (I) or a pharmaceutically acceptable acid addition salt thereof, are substituted imidazole derivatives of formula (IV) or addition salts thereof

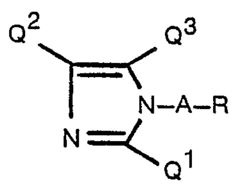

(IV)

wherein A and R are as defined in formula (I), and $Q^1$, $Q^2$ and $Q^3$ are the same or different, at least one being selected from thio (—SH), alkylthio (—Salkyl wherein alkyl has 1 to 4 carbon atoms) and halo preferably chloro or bromo, the remaining radical or radicals being selected from a radical having the same function or from hydrogen. The reaction conditions are chosen according to the nature of the radicals $Q^1$, $Q^2$ and $Q^3$. Desulphurisation may be performed by oxidative or reductive procedures using for example nitric acid or Raney nickel; and reductive dehalogenation by the use of zinc and acetic acid or Raney nickel or other reagents known in the literature.

Another class of examples include carboxyimidazoles or derivatives thereof of formula (VI):

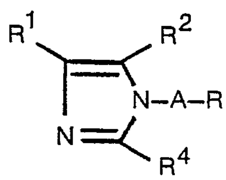

(VI)

wherein A and R are as defined in formula (I), at least one of $R^1$, $R^2$ and $R^4$ is carboxyl or a derivative thereof (for example an ester such as an alkyl ester, an acid halide such as the chloride, or the nitrile) and the other(s) is hydrogen or carboxyl or a derivative as described. The compounds of formula (VI) may be converted into the imidazoles of formula (I) by any suitable decarboxylation conditions which may simply comprise heating the compounds with or without a catalyst such as copper.

The imidazoles of formula (I) may also be made a compound of formula (VII):

(VII) 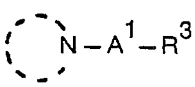 wherein 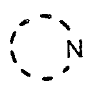

is 1-imidazolidine, 1-imidazole or 1-pyrazole, $A^1$ is a straight or branched saturated or unsaturated acyclic hydrocarbon radical, and $R^3$ is a cycloalkyl or cycloalkenyl radical of from 4 to 9 carbon atoms optionally substituted by alkyl as defined in formula (I), provided that at least one of

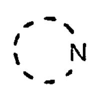,

$A^1$ and $R^3$ is other than 1-imidazole, a saturated acyclic hydrocarbon and an optionally substituted cycloalkyl group respectively as defined in formula (I). Thus an imidazoline (VIII):

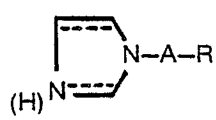

(VIII)

wherein one of---- represents an extra bond and A and R are defined in formula (I) may be dehydrogenated to the corresponding imidazole in the presence of a catalyst for example by heating to 250°C in the presence of palladium, nickel or platinum under pressure, or by heating with a dehydrogenating agent such as selenium or copper oxide. The 1-pyrazole compounds (VII) may be treated with ultra-violet irradiation, optionally under an inert atmosphere (e.g. argon) in for example 1,2-dimethoxyethane at room or elevated temperatures (see for example "Ring Transformations of

Heterocycles" edited van der Plas, Academic Press, 1973 at page 261). The unsaturated imidazoles of formula (I) (in formula (VII), $A^1$ and/or $R^3$ are unsaturated) may be reduced to the corresponding saturated compounds with a noble metal catalyst, for example platinum or palladium in alkanol.

The intermediates for use in the above described reactions may also be made by conventional methods known in the art. Thus the 1-pyrazole and 1-imidazoline intermediates (formula (VII) may be prepared by alkylation of pyrazole and imidazoline in an analogous manner to that described above for preparation of the corresponding imidazoles. The intermediates of formula (III) may be made in known manner preferably by halogenation of the corresponding alcohols (formula (III). Z = —OH) where in such compounds R is cycloalkenyl; the alcohol is conveniently prepared by the Prins reaction from the cycloalkene and paraformaldehyde (Bull. Chem. Soc. Japan 46/8, 2512—5, 1973). The substituted imidazole intermediates of formula (IV) may be made in known manner, for example see "Imidazole and its derivatives" Part I. Ed. K. Hofmann, Interscience Publishers In. New York, 1973. For example the 2-thioimidazoles of formula (IV) may be made by cyclisation of an acetal of formula (IX):

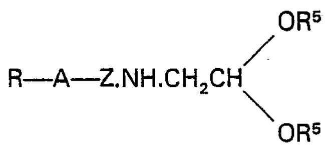

with thiocyanate, wherein $R^5$ is alkyl.

The pharmaceutically acceptable addition salts of the compounds of formula (I) may be prepared by any method known in the art. In particular they may be prepared by treating the parent imidazole with the appropriate acid.

Examples of the addition salts of the compounds of formula (I) include those salts derived from the following acids: oxalic, hydrochloric, hydrobromic, sulphuric, nitric, perchloric, fumaric, maleic, phosphoric, glycolic, lactic, salycyclic, succinic, toluene-*p*-sulphonic, tartaric, acetic, citric, methanesulphonic, formic, benzoic, malonic, naphthalene-2-sulphonic and benzene-sulphonic.

The imidazoles of formula (I) may be used in conjunction with a phosphodiesterase inhibitor, which provides a further, synergistic increase in effect, as it acts against platelet aggregation by a different pathway.

Suitable phosphodiesterase inhibitors for use in potentiating the anti-aggregatory effects of the active compounds include as such or as pharmaceutically acceptable salts:—

(a) Xanthine derivatives such as:—
Theophylline(3,7-dihydro-1,3-dimethyl-1*H*-purine-2,6-dione), and salts thereof.
3-Isobutyl-1-methyl-xanthine;
Caffeine(3,7-dihydro-1,3,7-trimethyl-1*H*-purine-2,6-dione) and salts thereof; and
Aminophylline (adduct of Theophylline and 1,2-ethanediamine (2:1)).

(b) Isoquinoline derivatives, for example:—
Papaverine(1-[(3,4-dimethoxyphenyl)methyl]-6,7-dimethoxyisoquinoline) and salts thereof; and
6,7-Diethoxy-1-(4,5-diethoxybenzyl)isoquinoline or its salts e.g. its hydrochloride;

(c) Derivatives of pyrimido(5,4-d)-pyrimidine, for example:—
Dipyridamole(2,2′,2″,2‴-(4,8-dipiperidinopyrimido[5,4-d]pyrimidin-2,6-diylnitrilo)tetraethanol) and its salts;
2,2′,2″,2‴-[[4-(1-piperidinyl)pyrimido[5,4-d]pyrimidin-2,6-diyl]dinitrilo]tetrakisethanol and its salts; and
2,4,6-tri-4-morpholinylpyrimido[5,4-d]pyrimidine and its salts.

(d) Derivatives of thieno[3,2-d]pyrimidine, for example:—
N-[4-(4-morpholinyl)thieno]3,2-d[pyrimidin-2-yl]-1,2-ethanediamine.

(e) Derivatives of pyrazolo[3′,4′:2,3]pyrido[4,5-b][1,5]benzodiazepin-6-(3*H*)-one, for example:—
3-Ethyl-7,12-dihydro-7,12-dimethylpyrazolo[4′,3′:5,6]pyrido[4,3-b]-[1,5]benzodiazepin-6-(3*H*)-one;
3-Ethyl-7,12-dihydro-9-methoxy-7,12-dimethylpyrazolo[3′,4′:2,3]pyrido[4,5-b][1,5]benzodiazepin-6-(3*H*)-one; and
10-Chloro-3-ethyl-7,12-dimethyl-7,12-dihydropyrazolo[4′,3′:5,6]pyrido[4,3-b][1,5]benzodiazepin-6-(3*H*)-one.

(f) Derivatives of 1*H*- or 2*H*-pyrazolo[3,4-b]pyridine, for example:—
4-(Butylamino)-1-ethyl-1*H*-pyrazolo[3,4-b]pyridine-5-carboxylic acid ethyl ester;
4-(Butylamino)-1*H*-pyrazolo[3,4-b]pyridine-6-carboxylic acid ethyl ester;
4-Chloro-1-ethyl-3-methyl-1*H*-pyrazolo[3,4-b]pyridine-5-acetonitrile;
1-Ethyl-4-(isopropylidenehydrazino)-3-methyl-1*H*-pyrazolo[3,4-b]pyridine-5-carboxylic acid ethyl ester or its salts such as its hydrochloride hemihydrate; and
2-Methyl-6-phenyl-4-(1-piperidinyl)-2*H*-pyrazolo[3,4-b]pyridine or its salts e.g. its hydrochloride.

(g) Derivatives of 5*H*-furo-[3,4-e]pyrazolo[3,4-b]pyridine-5-one, for example:—
4-(Butylamino)-1-ethyl-1,7-dihydro-7-hydroxy-5*H*-furo-[3,4-e]pyrazolo[3,4-b]pyridine-5-one;
and

(h) Derivatives of 1(2*H*)-naphthalenone, for example:—
2[(Dimethylamino)methyl]-3,4-dihydro-7-methoxy-1(2*H*)-naphthalenone or its salts e.g. its 1:1 hydrochloride.

The active compounds are particularly useful in the treatment and/or prophylaxis of thrombo-embolic disorders in mammals, including man. It is to be understood that the term "thrombo-embolic disorders" includes those disorders whose etiology is associated with platelet aggregation.

The active compounds are useful wherever it is desired to inhibit platelet aggregation and/or to reduce the adhesive character of platelets, and consequently to treat or prevent the formation of thrombi in mammals, including man. For example, the compounds are useful in the treatment and prevention of myocardial infarcts cerebro-vascular thrombosis and ischaemic peripheral vascular disease; to treat and prevent post-operative thrombosis; and to promote patency of vascular grafts following surgery.

The active compounds are also useful as an addition to blood, blood products, blood substitutes, and other fluids which are used in artificial extra-corporeal circulation and perfusion of isolated body portions, e.g., limbs and organs, whether attached to the original body, detached and being preserved or prepared for transplant, or attached to a new body. It may also be used in laboratory animals, e.g. cats, dogs, rabbits, monkey and rats, for these purposes in order to develop new methods and techniques for organ and limb transplants.

The active compounds also exhibit some vasodilatory action on blood vessels and therefore have a utility as anti-hypertensives for the treatment of high blood pressure in mammals, including man.

The amount of active compound required for therapeutic or prophylactic effect will vary with the route of administration, and the nature of the condition under treatment. In general a suitable dose for a mammal, including man, of active compound will lie in the range of 0.1 to 300 mg per kg body weight, particularly from 0.5 to 10 mg per kg body weight, for example 2 mg per kg. A suitable single oral dose for an adult human lies within the range of 50 to 600 mg, for example 150 mg given say three times a day.

While it is possible for the active compounds to be administered as the raw chemical it is preferable to present them as a pharmaceutical formulation. The formulations, both for veterinary and for human medical use, of the present invention comprise an active compound as above defined, together with one or more acceptable carriers therefor and optionally other therapeutic ingredients. The carrier(s) must be 'acceptable' in the sense of being compatible with the other ingredients of the formulation and not deleterious to the recipient thereof. It should be noted that the references herein to pharmaceutical formulations do not include mere mixtures consisting of the active compound a non-sterile organic solvent or water. Unit doses of a formulation may contain between 60 mg and 1.5 g of an active compound. For example, a tablet may contain 1-cyclooctylmethyl-imidazole or a pharmaceutically acceptable acid addition salt thereof in an amount of 50 to 500 mg based on the amount of imidazole base.

The formulations include those suitable for oral, rectal, vaginal or parenteral (including subcutaneous, intramuscular and intravenous) administration. Preferred formulations include tablets, capsules and injectable suspensions or solutions.

The formulations may conveniently be presented in unit dosage form and may be prepared by any of the methods well known in the art of pharmacy. All methods include the step of bringing into association the active compound (in the form of the base or a pharmaceutically acceptable acid addition salt) with the carrier which constitutes one or more accessory ingredients. In general the formulations are prepared by uniformly and intimately bringing into association the active compound with liquid carriers or finely divided solid carriers or both, and then, if necessary, shaping the product into the desired formulation.

The following Examples are provided by way of an illustration of the present invention and should in no way be construed as constituting a limitation thereof. All temperatures are given in degrees Celsius.

Example 1

1-Cyclooctylmethylimidazole

Imidazole (2.0 g, 0.03 mol) was added to a solution of sodium (0.7 g, 0.03 mol) in dry ethanol (50 ml). The mixture was stirred and heated to boiling when bromomethylcyclooctane (5.5 g, 0.027 mol) was added dropwise. Following the addition, the reaction mixture was stirred and boiled for 15 h.

After cooling, the reaction mixture was filtered, and the filtrate concentrated under reduced pressure. The residue was dissolved in 2*M*-hydrochloric acid (100 ml) and the solution washed with ether (25 ml). The aqueous layer was basified with 10*M*-sodium hydroxide solution and then extracted with chloroform (3 × 50 ml). The chloroform extracts were combined and dried ($MgSO_4$). Evaporation of the chloroform gave an oil which was purified using a silical gel column and elution with ethyl acetate/methanol (9:1). The product was further purified by distillation, b.p. 120—122°/0.2 mmHg.*.

By the method described in example 1 above the following compounds were prepared:

(a) 1-cyclooctylvinylimidazole
(b) 1-(2-cyclooctylethyl)imidazole
(c) 1,1-cyclooctyl)ethylimidazole
(d) 1-(3-cyclooctylpropyl)imidazole
(e) 1-(3-methylcyclohept-2-enylmethyl)imidazole
(f) 1-(3-methylcycloheptylmethyl)imidazole
(g) 1-(cyclohex-3-enylmethyl)-imidazole m.p. 36—37 degrees
(h) 1-(2-cyclohexylethyl)imidazole b.p. 148—150 degree 95—96 degrees/0.2 mm (0,267 × $10^{-8}$ Pa)
(i) 1-cyclobutylmethylimidazole b.p. 148—150 degrees/25 mm (33,325 × $10^{-8}$ Pa)

## Example 2

Preparation of 1-cyclopentylmethylimidazole

Imidazole (6.8 g, 0.1 mol) was added to a solution of sodium (2.3 g, 0.1 mol) in dry ethanol (100 ml). This solution was stirred and heated to reflux when bromomethylcyclopentane (16.3 g, 0.1 mol) was added dropwise. Following the addition, the mixture was stirred and heated under reflux for 16 h.

After cooling, the reaction mixture was filtered and the filtrate concentrated under pressure. The residue was dissolved in 2*M*-hydrochloric acid (150 ml) and the solution washed with ether. The aqueous solution was basified with 10*M*-sodium hydroxide solution, and the product extracted with chloroform (3 × 50 ml). The extracts were combined, dried ($MgSO_4$), and the solution concentrated to afford a yellow oil.

The oil was purified using a silica gel column and elution with ethyl acetate/methanol (9:1). The product fractions were combined and concentrated under reduced pressure to afford 1-cyclopentylmethylimidazole (1.9 g), which was further purified by distillation, b.p. 68—69°/0.125 mmHg (0,167 × $10^{-8}$ Pa).

## Example 3

Preparation of 1-(3-cyclopentylpropyl)imidazole

Imidazole (1.0 g, 0.0147 mol) was added to a solution of sodium (0.34 g, 0.0148 mol) in dry ethanol (30 ml). This solution was stirred and heated to boiling when 3-bromopropylcyclopentane (2.94 g, 0.0154 mol) was added dropwise. Following the addition, the reaction mixture was stirred and boiled for 20 h.

After cooling, the mixture was filtered and the filtrate concentrated under reduced pressure. The residue was dissolved in 2*M*-hydrochloric acid (50 ml) and the solution washed with ether (25 ml). The acid solution was then basified with 10*M*-sodium hydroxide solution, and the product extracted with chloroform (3 × 25 ml). The combined extracts were dried ($MgSO_4$) and concentrated under reduced pressure to afford a yellow oil (2.1 g).

The oil was purified by column chromatography (silica gel) using ethyl acetate/methanol (9:1) as eluent. The product fractions were combined and concentrated to afford 1-(3-cyclopentylpropyl)imidazole which was further purified by distillation, b.p. 89—90°/0.1 mmHg (0,133 × $10^{-8}$ Pa).

## Example 4

Preparation of 1-(cycloheptylmethyl)imidazole

Bromomethylcycloheptane (5.3 g, 0.0278 mol) was added dropwise to a stirred solution of potassium *t*-butoxide (3.1 g, 0.0277 mol) and imidazole (1.9 g, 0.0279 mol) in dry *n*-butanol (50 ml) maintained at 100° and under dry nitrogen. After the additoin (~20 mins) the temperature of the reaction mixture was raised to boiling. The reaction mixture was then stirred and boiled for 7 h and then cooled.

The mixture was filtered, and the *n*-butanol was removed under reduced pressure to give a pale yellow oil. The oil was dissolved in 2*M*-hydrochoric acid (100 ml) and the acid solution was washed with ether (100 ml) and then basified with 10*M*-sodium hydroxide solution and the resulting suspension was extracted with chloroform (3 × 50 ml). The chloroform extracts were combined, dried ($MgSO_4$), and concentrated under reduced pressure to give a pale yellow oil.

The oil was purified using a silica gel column and elution with ethyl acetate/methanol (9:1). Concentration of the fractions containing 1-(cycloheptylmethyl)imidazole gave a pale yellow oil which was further purified by distillation, b.p. 92—94°/0.1 mmHg (0,133 × $10^{-8}$ Pa).

## Example 5

Preparation of 1-(2-cyclooctenylmethyl)imidazole

(a) Preparation of 2-cyclooctene-1-methanol using the Prins reaction (Uchida *et al., Bull. Chem. Soc.*, Japan, 1973, *46*, 2512)

Cyclooctene (69.0 g, 0.63 mol) was added dropwise, to a stirred suspension of paraformaldehyde

(24.0 g) in 98% formic acid (100 ml). After the addition, the reaction mixture was stirred and heated under reflux for 2 h. Water (100 ml) was then added, and the aqueous solution was extracted with ether (50 ml). The ether solution was washed with saturated sodium bicarbonate solution (5 x 50 ml), with water (2 x 50 ml), and dried ($MgSO_4$). Concentration under reduced pressure afforded a brown oil which was purified by distillation, the fraction b.p. 80—110°/24 mmHg (31,992 x $10^{-8}$ Pa) being retained.

A portion of the aforesaid oil (10 g) was treated with Claisen's alkali [potassium hydroxide (10 g), methanol (31.2 ml) and water (8 ml)], and the reaction mixture was then boiled for 2 h.

After cooling, the mixture was poured onto iced-water (50 ml) and extracted with ether (3 x 50 ml). The ether extracts were combined and dried ($MgSO_4$). Concentration of the solution under reduced pressure afforded an oil which was distilled, to afford 2-cyclooctene-1-methanol, b.p. 128—130°/23 mmHg (30,659 x $10^{-8}$ Pa).

(b) Preparation of 2-cyclooctene-1-bromomethane

A solution of phosphorus tribromide (1.02 ml, 0.0105 mol) in petroleum ether (b.p. 40—60°, 5 ml) was added dropwise to a stirred solution of 2-cyclooctene-1-methanol (2.8 g, 0.02 mol) and dry pyridine (0.104 g, 0.0013 mol) in petroleum ether (b.p. 40—60°; 15 ml) at −10°. After the addition, the reaction mixture was set aside at ambient temperature for 48 h.

The reaction mixture was treated with water (50 ml) and the organic layer separated. The aqueous solution was extracted with petroleum ether (b.p. 40—60°,3 x 25 ml) and the organic layer and petroleum ether extracts combined, washed with 2*M*-sodium hydroxide solution (25 ml), and with water (25 ml), and then dried ($MgSO_4$). Concentration of the solution under reduced pressure gave an oil 2.3 g) which was distilled, b.p. 48—50°/0.25 mmHg (0,333 x $10^{-8}$ Pa).

(c) Preparation of 1-(2-cyclooctenylmethyl)imidazole

2-Cyclooctene-1-bromomethane (0.7 g, 0.0034 mol) was added dropwise to a boiling solution of imidazole (0.24 g, 0.0035 mol) and potassium *t*-butoxide (0.39 g, 0.0035 mol) in dry *n*-butanol, under dry nitrogen. After the addition, the reaction mixture was stirred and heated under reflux for 1 h. The pure product was obtained as described in Example 4, b.p. 108—110°/0.02 mmHg (0,0267 x $10^{-8}$ Pa).

## Example 6

Preparation of 1-(4-methylcyclohexylmethyl)imidazole

1-Bromomethyl-4-methylcyclohexane (3.1 g, 0.0162 mol) was added dropwise to a stirred, boiling solution of imidazole (1.12 g, 0.0165 mol) and potassium *t*-butoxide (1.85 g, 0.0165 mol) in dry *n*-butanol, under dry nitrogen. After the addition, the reaction mixture was stirred and heated under reflux for 10 h.

After cooling, the reaction mixture was filtered, and then concentrated under reduced pressure. The residue was dissolved in 2*M*-hydrochloric acid (100 ml) and the solution was washed with ether (50 ml). The acid solution was basified with 10*M*-sodium hydroxide solution and extracted with chloroform (3 x 50 ml). The combined chloroform extracts were dried ($MgSO_4$) and then concentrated under reduced pressure. The oily residue was purified using a silica gel column and elution with ethyl acetate/methanol (9:1). The fractions containing 1-(4-methylcyclohexylmethyl)imidazole were combined, concentrated, and the resulting oil distilled, b.p. 80°/0.125 mmHg (0,167 x $10^{-8}$ Pa).

## Example 7

Biological Results

Horse platelets were prepared from whole horse blood by differential centrifugation. Approximately $10^6$ platelets were homogenised in 1 ml 100 mM Tris buffer pH 7.4. Various concentrations of active compound were added and the reaction sets incubated for 5 minutes at ambient temperature. To each tube was added 20 nM of arachidonic acid containing $10^6$ DPM of labelled arachidonic acid and the tubes incubated for 3 minutes at 37°C in a shaking water bath. After incubation the radioactive products were extracted from the acidified aqueous phase wieth ethyl acetate and after concentration resolved by thin layer chromotography on silica gel with chloroform/methanol/acetic acid/water (90:8:1:0.8) as a developing solvent. The amount of thromboxane produced was measured by scraping the radioactive zone corresponding to thromboxane $B_2$ and estimating the radioactivity in a liquid scintillation counter.

The concentration of active compound to reduce the enzyme activity by 50% ($ED_{50}$) was established. The results are shown in Table A.

The selectivity of the active compounds was measured in a similar manner to that described above and the amount of PGE, PGF and PGD produced was determined. The greater the selectivity, the more of the prostaglandins are produced indicating lower inhibition of cyclo-oxygenase.

The $ED_{50}$ and Selectivity results are shown in Table A in which 0 indicates no selectivity; + low selectivity; ++ medium selectivity; and +++ high selectivity; ++++ exceptionally high selectivity.

TABLE A

| Compound (Reference Compound) | $ED_{50}$ μg/ml | Selectivity |
|---|---|---|
| (Imidazole) | ⩾500 | 0 to + |
| (1-Methylimidazole) | ⩾200 | ++ |
| 1-Cyclopentylmethylimidazole | ~5 | +++ |
| 1-Cyclohexylethylimidazole | 4 | +++ |
| 1-Cyclooctylmethylimidazole | 4 | +++ |
| 1-Cyclohex-3-enylmethylimidazole | ~5 | +++ |
| 1-Cyclobutylmethylimidazole | 50 | +++ |
| 1-Cycloheptylmethylimidazole | 4.7 | ++++ |
| 1-(4-Methylcyclohexylmethyl)imidazole | 6.6 | +++ |

Example 8

*Tablet formulation*

| | |
|---|---|
| 1-Cyclooctylmethylimidazole (as a salt) | 150 mg |
| Starch | 25 mg |
| Polyvinylpyrrolidone | 2 mg |
| Magnesium stearate | 3 mg |

The imidazole salt is ground to a fine powder, blended with the starch and then the mixture granulated with an aqueous solution of the polyvinylpyrrolidone. The granules are sieved 1000 μ, dried, sieved again and the magnesium stearate added. The mixture is then compressed into tablets.

In the same manner, tablets of 1-cyclohex-3-enylmethylimidazole and 1-cyclohexylethylimidazole are prepared.

Example 9

*Tablet formulation*

Tablets (150 mg) of the imidazoles described in the preceding example are prepared as in the same manner from the following ingredients:

| | |
|---|---|
| The Imidazole Compound (as a salt) | 150 mg |
| Lactose | 100 mg |
| Starch | 30 mg |
| Polyvinylpyrrolidone | 2 mg |
| Magnesium stearate | 3 mg |

In the preparation, the lactose is blended with the starch.

Example 10

*Tablet formulation*

Tablets (100 mg) of the imidazoles of Example 8 are prepared in the same manner from the following ingredients:

| | |
|---|---|
| The Imidazole Compound (as a salt) | 100 mg |
| Sodium starch glycolate | 10 mg |
| Polyvinylpyrrolidone | 2 mg |
| Magnesium stearate | 3 mg |

Example 11

*Tablet formulation*

Tablets (150 mg) of the imidazoles of Example 8 are prepared in the same manner from the following ingredients, except that the starch, pregelled starch and imidazole compound are all blended together prior to granulation:

| | |
|---|---|
| The Imidazole Compound (as a salt) | 150 mg |
| Starch | 25 mg |
| Pregelled starch | 5 mg |
| Magnesium stearate | 3 mg |

Example 12

*Injectable formulation*

| | |
|---|---|
| Imidazole compound of formula (I) | 15.0 g |
| Lactic Acid B.P. | q.s. to pH 3.0 |
| Water for Injections B.P. | to 100.0 ml |

Suspend the compound in $\frac{3}{4}$ of the available quantity of water. Add sufficient Lactic Acid to dissolve the compound and to reduce the pH to 3.0. Dilute to volume with Water for Injections.

Sterilise the solution by passage through a membrane filter, pore size 0.22 $\mu$m.

Distribute the solution using aseptic precautions into sterilised ampoules, 1 ml per ampoule. Seal by fusion of the glass.

Each 1 ml ampoule supplies 150 mg of the imidazole compound: 1-cyclooctylmethylimidazole fumarate.

Example 13

*Injectable formulation*

| | |
|---|---|
| Imidazole compound of formula (I) | 15.0 g |
| Citric Acid B.P. | q.s. to pH 3.0 |
| Chlorocresol | 0.1 g |
| Water for Injections to | 100.0 ml |

Suspend the compound in $\frac{1}{2}$ the final volume of Water for Injections. Add sufficient Citric Acid as a 10% solution in Water for Injections to dissolve the compound and reduce the pH to 3.0. Dilute to volume with Water for Injections.

Sterilise the solution by passage through a membrane filter, pore size 0.22 $\mu$m.

Distribute the solution with aseptic precautions into sterilised vials, 25 ml per vial. Stopper with sterile rubber closures and seal with an aluminium cap.

Each 1 ml of solution provides 150 mg of the compound: 1-cyclooctylmethylimidazole fumarate.

Example 14

*Injectable formulation*

In the manner described in the preceding two Examples, injectable formulations of 1-cyclohexylethylimidazole and 1-cyclohex-3-enylmethylimidazole salts were prepared.

Example 15

(a) 1-*n*-Butylimidazole

1-Bromobutane (27.4 ml, 0.255 mol) was added dropwise to a stirred solution of imidazole (13.6 g, 0.2 mol) in a mixture of methanol (30 ml) (30 ml) and 10*M*-sodium hydroxide solution (30 ml) maintained at 30—40°. Following the addition, the reaction mixture was stirred and heated under reflux for 12 h.

After concentration under reduced pressure, the residue was extracted with chloroform (2 x 50 ml) and the extracts were combined and dried ($MgSO_4$). Concentration of the extracts under reduced pressure gave a yellow oil which was purified using a silica gel column and ethyl acetate/methanol (9:1) as eluent. The product fractions were combined, concentrated, and the resulting oil distilled to afford 1-*n*-butylimidazole, b.p. 122—123°/20 mm Hg (26,666 x $10^{-8}$ Pa).

(b) 1-*n*-Butylimidazole oxalate

To 1-*n*-butylimidazole (0.31 g, 0.0025 mol) in methanol (5 ml) was added oxalic acid (0.225 g, 0.0025 mol) in methanol (5 ml). After heating under reflux for 5 minutes, the mixture was evaporated under reduced pressure to afford a white solid. Recrystallisation of the solid from ethanol/ether (1:4) afforded 1-*n*-butylimidazole oxalate as colourless needles, m.p. 82—83°.

(c) 1-*n*-Butylimidazole perchlorate

To 1-*n*-butylimidazole (0.31 g, 0.0025 mol) in methanol (5 ml) was added perchloric acid (0.33 g, 70%) in methanol (3 ml). After boiling for 5 minutes, the mixture was evaporated under reduced pressure to afford a white solid. Recrystallisation of the solid from ethyl acetate/ether (1:4) afforded 1-*n*-butylimidazole perchlorate as a white solid, m.p. 54—55°.

Example 16

By the method described in Example 15 above the following compound was prepared:—

1-(3-Methylbutyl)imidazole, b.p. 62—64°/0.2 mmHg (0,2666 x $10^{-8}$ Pa). In place of sodium hydroxide, sodium bicarbonate was used.

Example 17

1-But-2-enylimidazole (1-crotylimidazole)

1-Chlorobut-2-ene (3.2 g, 0.035 mol) in methanol (35 ml) was added dropwise to a stirred, boiling mixture of imidazole (11.9 g, 0.175 mol) and sodium bicarbonate (2.95 g, 0.035 mol) in methanol (35 ml). After the addition, the reaction mixture was stirred and boiled for 24 hr.

After cooling, the reaction mixture was filtered, and the solution concentrated under reduced pressure. The residue was dissolved in water (60 ml) and the product extracted with chloroform (3 x 20 ml). The combined chloroform extracts were washed with saturated brine (25 ml), dried ($MgSO_4$), and then concentrated under reduced pressure. Distillation of the resulting oil gave 1-but-2-enylimidazole as a colourless oil, b.p. 123—124°/18 mmHg (23,994 x $10^{-8}$ Pa).

Example 18

(a) 1-*n*-Pentylimidazole (1-*n*-Amylimidazole)

1-*n*-Bromopentane (30.0 ml, 0.24 mol) was added dropwise to a stirred solution of imidazole (13.6 g, 0.2 mol) in methanol (30 ml) and 10*M*-sodium hydroxide (30 ml). Following the addition, the reaction mixture was stirred and heated under reflux for 24 h.

After cooling, the solution was concentrated under reduced pressure and the residue extracted with chloroform (2 x 50 ml). The chloroform extracts were combined, dried ($MgSO_4$), and concentrated to afford a yellow oil which on distillation gave 1-*n*-pentylimidazole, b.p. 66—68°/0.15 mmHg.

(b) By the method described in (a) the following compound was prepared:—

1-*n*-Hexylimidazole, b.p. 78—80°/0.2 mmHg (0,2666 x $10^{-8}$ Pa).

Example 19

1-Cyclohexylmethylimidazole

Bromomethylcyclohexane (39.8 g, 0.225 mol) was added dropwise to a stirred, boiling solution of imidazole (13.6 g, 0.2 mol) and sodium (4.6 g, 0.2 mol) in dry ethanol (100 ml). Following the addition, the reaction mixture was stirred and boiled for 5 h.

After cooling, the reaction mixture was filtered, and the filtrate concentrated under reduced pressure. The residue was dissolved in water (150 ml), washed with ether (50 ml), and the product extracted with chloroform (3 x 50 ml). The combined chloroform extracts were dried ($MgSO_4$) and

concentrated to give a yellow oil. Distillation of the oil gave 1-cyclohexylmethylimidazole, b.p. 86—90°/0.2 mmHg (0,2666 x $10^{-8}$ Pa) m.p. 27—30°.

Example 20

1-Cycloheptylimidazole

Bromocycloheptane (10.0 g, 0.0565 mol) was added dropwise to a stirred, boiling mixture of imidazole (3.85 g, 0.0566 mol) and sodium bicarbonate (4.75 g, 0.0565 mol) in methanol. Following the addition, the reaction mixture was stirred and boiled for 24 h.

After cooling, the mixture was concentrated under reduced pressure. The residue was dissolved in 2*M*-hydrochloric acid (150 ml), and this solution was washed with ether (50 ml). The acid solution was basified with 10*M*-sodium hydroxide solution, and then extracted with chloroform (3 x 50 ml). The combined extracts were dried ($MgSO_4$) and then concentrated. The resulting oil was purified on a silica gel column and elution with ethyl acetate/methanol (9:1). The product fractions were pooled, concentrated, and the resulting oil was distilled, b.p. 102—104°/0.15 mmHg (0,19995 x $10^{-8}$ Pa) m.p. 31—35°.

Example 21

Preparation of 1-cyclopentylimidazole

To a stirred soluton of imidazole (6.8 g, 0.1 mol) in dry dimethylformamide (100 ml), under dry nitrogen, was added sodium hydride (4.8 g, 50% dispension in oil, 0.1 mol) portionwise. After the addition, the reaction mixture was stirred for 0.25 h when bromocyclopentane (14.9 g, 0.1 mol) in dry dimethylformamide (100 ml) was added dropwise. The reaction mixture was then stirred at ambient temperature for 1 h, after which it was poured onto an equal volume of water and this mixture was shaken well. The product was extracted with chloroform (3 x 200 ml) and the extracts combined and dried ($MgSO_4$). The solution was concentrated under reduced pressure to afford a yellow oil. Purification by chromatography on silica gel and elution with methanol/ethyl acetate (1:9) followed by distillation gave the product, b.p. 67—68°/0.2 mm (0,2666 x $10^{-8}$ Pa).

Example 22

Preparation of 1-pent-2-enylimidazole

1-Bromopent-2-ene (18.5 g, 0.124 mol) was added dropwise to a stirred, boiling mixture of imidazole (10.0 g, 0.147 mol) and sodium bicarbonate (12.4 g, 0.015 mol) in dry methanol (50 ml). Following the addition, the mixture was stirred and heated under reflux for 24 h.

After cooling, the reaction mixture was filtered and the filtrate concentrated under reduced pressure. The residue was dissolved in 2*M*-hydrochloric acid and this solution was washed with ether. The acid solution was basified with 10*M*-sodium hydroxide solution and the product was extracted with chloroform (3 x 50 ml). The combined extracts were dried ($MgSO_4$) and then concentrated to afford an oil which was purified on a silica gel column by elution with ethyl acetate/methanol (9:1). The product fractions were pooled and concentrated to give 1-pent-2-enylimidazole, which was further purified by distillation, b.p. 134°/15 mmHg (19,995 x $10^{-8}$ Pa).

Example 23

Preparation of 1-pent-4-enylimidazole

1-Bromopent-4-ene (10.0 g, 0.067 mol) was added dropwise to a stirred, boiling mixture of imidazole (9.0 g, 0.132 mol) and sodium bicarbonate (11.0 g, 0.13 mol) in methanol (50 ml). Following the addition, the mixture was stirred and heated under reflux for 24 h.

After cooling, the reaction mixture was filtered and the filtrate concentrated under reduced pressure. The residue was dissolved in 2*M*-hydrochloric acid and this solution was washed with ether. The acid solution was basified with 10*M*-sodium hydroxide solution and the product was extracted with chloroform (3 x 50 ml). The combined extracts were dried ($MgSO_4$) and then concentrated to afford an oil which was purified on a silica gel column and elution with ethyl acetate/methanol (9:1). The product fractions were pooled and concentrated to give 1-pent-4-enylimidazole, which was further purified by distillation, b.p. 132°/12 mmHg. (15,996 x $10^{-8}$ Pa).

Example 24

By the method described in the Biological Results in Example 7 above, the $ED_{50}$ values were obtained for the compounds set out in Table 1.

TABLE I

| Active compound (Reference compound) | $ED_{50}$ $\mu g/ml$ |
|---|---|
| (Imidazole) | ≥500 |
| (1-methylimidazole) | ≥200 |
| 1-n-Butylimidazole | 10 |
| 1-n-Pentylimidazole | 7 |
| 1-n-Hexylimidazole | 10 |
| 1-Pent-2-enylimidazole | 10 |
| 1-Pent-4-enylimidazole | 23 |
| 1-(3-Methylbutyl)imidazole | 20 |
| 1-Cyclohexylmethylimidazole | 2,5 |
| 1-Cycloheptylimidazole | ~6 |
| 1-Cyclopentylimidazole | 125 |

Example 25

*Tablet formulations*

By the methods described in the Tablet formulation Examples described above, tablets were prepared of:—

1-n-butylimidazole
1-(3-methylbutyl)imidazole
1-crotylimidazole
1-n-pentylimidazole
1-cyclohexylmethylimidazole
1-cyclopentylimidazole
1-cycloheptylimidazole
1-(pent-2-enyl)imidazole
1-(pent-4-enyl)imidazole.

In each case the imidazole was present as a salt, e.g. the fumarate or perchlorate.

Example 26

*Injectable formulations*

Injectable formulations were prepared by the method of the Injectable formulation in Example 12 above using the imidazoles mentioned in the immediately preceding Example.

Example 27

Salts of 1-Cyclooctylmethylimidazole

*A. Hydrogen Fumarate*

A solution of fumaric acid (0.22 g) in hot ethanol (10 ml) was added to a solution of 1-cyclooctylmethylimidazole (0.38 g) in ethanol (4 ml). After boiling for 10 minutes the solution was evaporated to afford a white solid. Recrystallisation from ethyl acetate afforded 1-cyclooctylmethylimidazole hydrogen fumarate (0.42 g) as white needles, m.p. 147—148°.

*B. Hydrogen Succinate*

A solution of succinic acid (0.23 g) in ethanol (~5 ml) was added to a solution of 1-cyclooctylmethylimidazole (0.38 g) in ethanol (5 ml). Evaporation of the solution afforded a white solid. Recrystallisation of the solid from ethyl acetate afforded 1-cyclooctylmethylimidazole hydrogen succinate (0.27 g) as colourless plates, m.p. 86—87°.

*C. Oxalate*

A solution of oxalic acid (0.17 g) and 1-cyclooctylmethylimidazole (0.38 g) in ethanol (20 ml) was boiled for 0.25 h, when evaporation of the solution afforded a white solid. Recrystallisation of the solid from ethyl acetate/ethanol/petroleum ether (b.p. 40—60°C) afforded 1-cyclooctylmethylimidazole oxalate as white needles, m.p. 141—142°.

*D. Hydrochloride*

1-Cyclooctylmethylimidazole (~0.3 g) was dissolved in dry ether (30 ml), when a stream of dry hydrogen chloride was passed through the solution at −20°C. The resulting white precipitate was filtered off under dry nitrogen and recrystallised from ethyl acetate/petroleum ether (b.p. 40—60°) to afford 1-cyclooctylmethylimidazole hydrochloride as a white solid, m.p. 20—22°C.

Example 28

Preparation of 1-Cyclohexylimidazole

*A. Cyclohexylaminoacetaldehyde diethyl acetal*

A mixture of chloroacetaldehyde diethyl acetal (42.5 g) and cyclohexylamine (139.0 g) was stirred and heated under reflux for 4 hr. After cooling, water (150 ml) was added and the solution basified with sodium hydroxide solution (10 M). The resulting suspension was extracted with ether (3 x 200 ml) and the extracts dried over magnesium sulphate. Evaporation of the ether gave a brown oil. Distillation of the oil *in vacuo* afforded cyclohexylaminoacetaldehyde diethyl acetal, 38.52 g, b.p. 66—72°/0.15 mmHg (0,19995 x $10^{-8}$ Pa).

*B. 1-Cyclohexyl-2-thioimidazole*

A mixture of cyclohexylaminoacetaldehyde diethyl acetal (21.5 g) and potassium thiocyanate (8.9 g) in 50% aqueous ethanol (50 ml) and concentrated hydrochloric acid (10 ml) was heated in an open flask on a steam bath for 3.5 hr. The resulting crystalline mass was dissolved in sodium hydroxide solution (~250 ml, 2M), the solution treated with charcoal, boiled and filtered. This solution was acidified with concentrated hydrochloric acid to afford the product as a white solid. Recrystallisation of the solid from ethyl acetate afforded 1-cyclohexyl-2-thioimidazole (13.2 g) as white plates, m.p. 181—182°.

*C. 1-Cyclohexylimidazole*

A mixture of 1-cyclohexyl-2-thioimidazole (2.0 g) and W2 Raney Nickel (~4.0 g), in ethanol (~25 ml) and ammonia solution (10 ml, 0.88) was vigorously stirred and heated under reflux for 2.0 hr. The catalyst was filtered off, and the solution evaporated to afford a white solid. Recrystallisation of the solid from petroleum ether (b.p. 60—80°) gave 1-cyclohexylimidazole (0.9 g) as white needles, m.p. 37—38°.

Example 29

By the method described in Example 1 above the following compounds were prepared:—

(a) 1-cyclooctylvinylimidazole
(b) 1-(2-cyclooctylethyl)imidazole
(c) 1-cyclooctylethylimidazole
(d) 1-(3-cyclooctylpropyl)imidazole
(e) 1-(3-methylcyclohept-2-enymethyl)imidazole
(f) 1-(3-methylcycloheptylmethyl)imidazole
(g) 1-(cyclohex-3-enylmethyl)imidazole m.p. 36—37°
(h) 1-cyclohexylethylimidazole b.p. 95—96°/0.2 mm (0,2666 x $10^{-8}$ Pa)
(i) 1-cyclobutylmethylimidazole b.p. 148—150°/25 mm (33,325 x $10^{-8}$ Pa)
(j) 1-cyclononylmethylimidazole

**Claims**

1. A pharmaceutical formulation, adapted for administration to a human or animal subject, comprising an imidazole of the formula

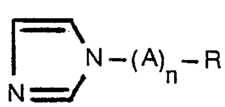

(I)

in which A is a straight or branched, saturated or unsaturated acyclic hydrocarbon radical of from 1 to 3 carbon atoms, n is 0 or 1, and R is a cycloalkyl or cycloalkenyl radical of from 4 to 9 carbon atoms, optionally substituted by one or more alkyl radicals each containing from 1 to 4 carbon atoms, or $(A)_n$ and R together form a straight-chain alkyl radical of from 4 to 7 carbon atoms or an alkenyl or alkynyl radical of from 4 to 9 carbon atoms, the imidazole being the free base of a pharmaceutically acceptable salt thereof, together with a pharmaceutically acceptable carrier therefor.

2. A pharmaceutical formulation as claimed in claim 1 characterised in that n is 1, A is a straight or branched, saturated or unsaturated acyclic hydrocarbon radical of from 1 to 3 carbon atoms and R is a cycloalkyl or cycloalkenyl radical of from 4 to 9 carbon atoms optionally substituted by one or more alkyl radicals each containing from 1 to 4 carbon atoms, the imidazole being the free base or a pharmaceutically acceptable salt thereof, together with a pharmaceutically acceptable carrier therefor.

3. A pharmaceutical formulation as claimed in claim 1 characterised in that n is 1, R is selected from a cycloalkyl and a cycloalkenyl radical of from 6 to 8 carbon atoms, and A is $—CH_2—$ or $—(CH_2)_2$.

4. A pharmaceutical formulation as claimed in claim 1 characterised in that the imidazole is selected from 1-cyclohexylethyl-imidazole, and 1-cyclohex-3-enylmethylimidazole, and pharmaceutically acceptable acid addition salts thereof.

5. A pharmaceutical formulation as claimed in any of claims 1 to 4 characterised in that it is in the form of a tablet, capsule or parenterally acceptable injectable solution or suspension.

6. A pharmaceutical formulation as claimed in claim 1 comprising 1-cyclohexylmethyl-imidazole or a pharmaceutically acceptable acid addition salt thereof, in association with a pharmaceutically acceptable carrier therefor.

7. A pharmaceutical formulation as claimed in claim 1 comprising 1-cyclohexylmethyl-imidazole or a pharmaceutically acceptable acid addition salt thereof in the form of a tablet, capsule or parenterally acceptable injectable solution or suspension.

8. A pharmaceutical formulation as claimed in claim 1 comprising 1-cyclooctylmethyl-imidazole or a pharmaceutically acceptable acid addition salt thereof, in association with a pharmaceutically acceptable carrier therefor.

9. A pharmaceutical formulation as claimed in claim 1 comprising 1-cyclooctylmethyl-imidazole or a pharmaceutically acceptable salt thereof in the form of a tablet, capsule or parenterally acceptable injectable solution or suspension.

10. A tablet as claimed in claim 9 characterised in that it contains from 50 to 500 mg of the imidazole or a pharmaceutically acceptable salt thereof, said amount being the amount of the imidazole base present.

11. A method of preparing formulations as claimed in claim 1 characterised in that one admixes an imidazole or a pharmaceutically acceptable acid addition salt as defined in claim 1 with a pharmaceutically acceptable carrier therefor.

12. An imidazole characterised in that it is of formula

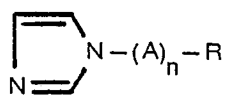

in which n is 0 and R is a cycloalkyl or cycloalkenyl radical or from 4 to 9 carbon atoms and optionally substituted by one or more alkyl radicals each containing from 1 to 4 carbon atoms, with the proviso that R is not unsubstituted cyclohexyl, or a pharmaceutically acceptable acid addition salt thereof.

13. An imidazole as claimed in claim 12 with the further proviso that R is not substituted cyclohexyl.

14. An imidazole characterised in that it is of formula

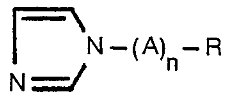

in which $n = 1$ and A and R together form an alkenyl radical of from 5 to 9 carbon atoms, or a pharmaceutically acceptable acid addition salt thereof.

15. An imidazole according to claim 12 which is 1-cyclopentylimidazole or 1-cycloheptylimidazole, or a pharmaceutically acceptable acid addition salt thereof.

16. An imidazole according to claim 14 which is 1-(pent-2-enyl)imidazole or 1-(pent-4-enyl)-imidazole, or a pharmaceutically acceptable acid addition salt thereof.

17. A pharmaceutical composition which comprises a compound according to any one of claims 12 to 16, and a pharmaceutically acceptable carrier therefor.

18. An imidazole of formula (I), as defined in claim 1, the imidazole being the free base or a pharmaceutically acceptable salt thereof, for use in the treatment or prophylaxis of thrombo-embolic disorders of a mammal or mammalian tissue.

19. An imidazole according to claim 18 characterised in that n is 1, A is a straight or branched, saturated or unsaturated acyclic hydrocarbon radical of from 1 to 3 carbon atoms and R is a cycloalkyl or cycloalkenyl radical of from 4 to 9 carbon atoms optionally substituted by one or more alkyl radicals each containing from 1 to 4 carbon atoms.

20. An imidazole according to claim 18 in which $(A)_n$ and R together form an alkenyl radical of from 5 to 9 carbon atoms.

21. An imidazole according to claim 18 in which $n = 0$.

22. 1-Cyclooctylmethylimidazole or a pharmaceutically acceptable acid addition salt thereof, for use in the treatment or prophylaxis of thrombo-embolic disorders in a mammal.

23. 1-Cyclooctylmethylimidazole or a pharmaceutically acceptable acid addition salt thereof for use in the treatment or prophylaxis of thrombi in a mammal.

24. 1-Cyclooctylmethylimidazole or a pharmaceutically acceptable acid addition salt thereof for use in the treatment or prophylaxis of myocardial infarcation in a mammal.

25. 1-Cyclohexylmethylimidazole or a pharmaceutically acceptable acid addition salt thereof for use in the treatment or prophylaxis of thrombo-embolic disorders in a mammal.

26. A pharmaceutical formulation according to any one of claims 1 to 10 for use in the treatment or prophylaxis of a thrombo-embolic disorder in a mammal.

**Revendications**

1. Formulation pharmaceutique se prêtant à l'administration à un sujet humain ou animal, qui comprend un imidazole de formule:

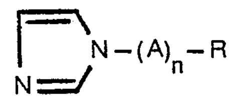

(I)

où A est un radical hydrocarboné acyclique saturé ou insaturé en chaîne droite ou ramifiée de 1 à 3 atomes de carbone, n vaut 0 ou 1 et R est un radical cycloalkyle ou cyclo-alcényle de 4 à 9 atomes de carbone éventuellement substitué par un ou plusieurs radicaux alcoyle comptant chacun 1 à 4 atomes de carbone, ou $(A)_n$ et R forment ensemble un radical alcoyle en chaîne droite de 4 à 7 atomes de carbone ou un radical alcényle ou alcynyle de 4 à 9 atomes de carbone, l'imidazole étant à l'état de base libre ou de sel pharmaceutiquement acceptable, en association avec un excipient pharmaceutiquement acceptable.

2. Formulation pharmaceutique suivant la revendication 1, caractérisée en ce que n vaut 1, A est un radical hydrocarboné acyclique saturé ou insaturé en chaîne droite ou ramifiée de 1 à 3 atomes de carbone et R est un radical cyclo-alcoyle ou cyclo-alcényle de 4 à 9 atomes de carbone éventuellement substitué par un ou plusieurs radicaux alcoyle comptant chacun 1 à 4 atomes de carbone, l'imidazole étant à l'état de base libre ou de sel pharmaceutiquement acceptable, en association avec un excipient pharmaceutiquement acceptable.

3. Formulation pharmaceutique suivant la revendication 1, caractérisée en ce que n vaut 1, R est choisi entre un radical cyclo-alcoyle et un radical cyclo-alcényle de 6 à 8 atomes de carbone et A représente $-CH_2-$ ou $-(CH_2)_2-$.

4. Formulation pharmaceutique suivant la revendication 1, caractérisée en ce que l'imidazole est choisi entre le 1-cyclohexyléthylimidazole et le 1-cyclohex-3-énylméthylimidazole, et leurs sels d'addition d'acides pharmaceutiquement acceptables.

5. Formulation pharmaceutique suivant l'une quelconque des revendications 1 à 4, caractérisée en ce qu'elle est présenté sous forme de comprimé, de capsule ou de solution ou suspension injectable parentéralement acceptable.

6. Formulation pharmaceutique suivant la revendication 1, qui comprend du 1-cyclohexylméthylimidazole ou un de ses sels d'addition d'acides pharmaceutiquement acceptables, en association avec un excipient pharmaceutiquement acceptable.

7. Formulation pharmaceutique suivant la revendication 1, qui comprend du 1-cyclohexylméthylimidazole ou un de ses sels d'addition d'acides pharmaceutiquement acceptables sous la forme d'un comprimé, d'une capsule ou d'une solution ou suspension injectable parentéralement acceptable.

8. Formulation pharmaceutique suivant la revendication 1, qui comprend du 1-cyclo-octylméthylimidazole ou un de ses sels d'addition d'acides pharmaceutiquement acceptables, en association avec un excipient pharmaceutiquement acceptable.

9. Formulation pharmaceutique suivant la revendication 1, qui comprend du 1-cyclo-octylméthylimidazole ou un de ses sels pharmaceutiquement acceptables sous la forme d'un comprimé, d'une capsule ou d'une solution ou suspension injectable parentéralement acceptable.

10. Comprimé suivant la revendication 9, caractérisé en ce qu'il contient de 50 à 500 mg de l'imidazole ou d'un sel pharmaceutiquement acceptable de celui-ci, cette quantité étant la quantité de l'imidazole base libre en présence.

11. Procédé de préparation de formulations suivant la revendication 1, caractérisé en ce qu'on mélange un imidazole ou un sel d'addition d'acide pharmaceutiquement acceptable comme défini dans la revendication 1 avec un excipient pharmaceutiquement acceptable.

12. Imidazole, caractérisé en ce qu'il répond à la formule:

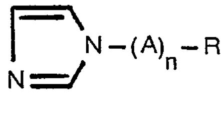

où n vaut 0 et R est un radical cyclo-alcoyle ou cyclo-alcényle de 4 à 9 atomes de carbone éventuellement substitué par un ou plusieurs radicaux alcoyle comptant chacun 1 à 4 atomes de carbone, étant

entendu que R n'est pas un radical cyclohexyle non substitué, ou un de ses sels d'addition d'acides pharmaceutiquement acceptables.

13. Imidazole suivant la revendication 12, étant entendu en outre que R n'est pas un radical cyclohexyle substitué.

14. Imidazole, caractérisé en ce qu'il répond à la formule

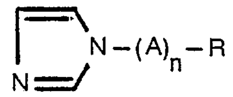

où n vaut 1 et A et R forment ensemble un radical alcényle de 5 à 9 atomes de carbone, ou un de ses sels d'addition d'acides pharmaceutiquement acceptables.

15. Imidazole suivant la revendication 12, qui est le 1-cyclopentylimidazole ou le 1-cycloheptylimidazole, ou un de leurs sels d'addition d'acides pharmaceutiquement acceptables.

16. Imidazole suivant la revendication 14, qui est le 1-(pent-2-ényl)imidazole ou le 1-(pent-4-ényl)imidazole, ou un de leurs sels d'addition d'acides pharmaceutiquement acceptables.

17. Composition pharmaceutique, qui comprend un composé suivant l'une quelconque des revendications 12 à 16 et un excipient pharmaceutiquement acceptable.

18. Imidazole de formule (I) suivant la revendication 1, qui est présenté à l'état de base libre ou de sel pharmaceutiquement acceptable, à utiliser pour le traitement ou la prophylaxie d'affections thromboemboliques chez un mammifère ou dans un tissu de mammifère.

19. Imidazole suivant la revendication 18, caractérisé en ce que n vaut 1, A est un radical hydrocarboné acyclique saturé ou insaturé en chaîne droite ou ramifiée de 1 à 3 atomes de carbone et R est un radical cyclo-alcoyle ou cyclo-alcényle de 4 à 9 atomes de carbone éventuellement substitué par un ou plusieurs radicaux alcoyle comptant chacun 1 à 4 atomes de carbone.

20. Imidazole suivant la revendication 18, dans lequel $(A)_n$ et R forment ensemble un radical alcényle de 5 à 9 atomes de carbone.

21. Imidazole suivant la revendication 18, dans lequel n vaut 0.

22. Le 1-cyclo-octylméthylimidazole ou un de ses sels d'addition d'acides pharmaceutiquement acceptables, à utiliser pour le traitement ou la prophylaxie d'affections thrombo-emboliques chez un mammifère.

23. Le 1-cyclo-octylméthylimidazole ou un de ses sels d'addition d'acide pharmaceutiquement acceptables, à utiliser pour le traitement ou la prophylaxie du thrombus chez un mammifère.

24. Le 1-cyclo-octylméthylimidazole ou un de ses sels d'addition d'acides pharmaceutiquement acceptables, à utiliser pour le traitement ou la prophylaxie de l'infarctus du myocarde chez un mammifère.

25. Le 1-cyclohexylméthylimidazole ou un de ses sels d'addition d'acides pharmaceutiquement acceptables, à utiliser pour le traitement ou la prophylaxie d'affections thrombo-emboliques chez un mammifère.

26. Formulation pharmaceutique suivant l'une quelconque des revendications 1 à 10, à utiliser pour le traitement ou la prophylaxie d'affections thrombo-emboliques chez un mammifère.

**Patentansprüche**

1. Pharmazeutische Formulierung, die zur Verabfolgung an einen Menschen oder ein Tier angepaßt ist, enthaltend ein Imidazol der Formel

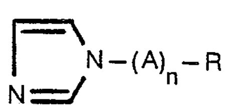

(I)

worin

A ein gerader oder verzweigter, gesättigter oder ungesättigter acylischer Kohlenwasserstoffrest mit

1 bis 3 Kohlenstoffatomen ist,

n für 0 oder 1 steht, und

R einen Cycloalkyl- oder Cycloalkenylrest mit 4 bis 9 Kohlenstoffatomen, der gegebenenfalls durch einen oder mehrere Alkylreste, die jeweils 1 bis 4 Kohlenstoffatome enthalten, substituiert ist, oder $(A)_n$ und R zusammen einen geradkettigen Alkylrest mit 4 bis 7 Kohlenstoffatomen oder einen Alkenyl- oder Alkinylrest mit 4 bis 9 Kohlenstoffatomen bilden, wobei das Imidazol die freie Base oder ein pharmazeutisch verträgliches Salz davon ist, zusammen mit einem pharmazeutisch unbedenklichen Träger dafür.

2. Pharmazeutische Formulierung nach Anspruch 1, dadurch gekennzeichnet, daß n gleich 1 ist, A einen geraden oder verzweigten, gesättigten oder ungesättigten acyclischen Kohlenwasserstoffrest mit 1 bis 3 Kohlenstoffatomen darstellt und R einen Cycloalkyl- oder Cycloalkenylrest mit 4 bis 9 Kohlenstoffatomen bedeutet, der gegebenenfalls durch ein oder mehrere Alkylreste substituiert ist, die jeweils 1 bis 4 Kohlenstoffatome enthalten, wobei das Imidazol die freie Base oder ein pharmazeutisch verträgliches Salz davon ist, zusammen mit einem pharmazeutisch unbedenklichen Träger dafür.

3. Pharmazeutische Formulierung nach Anspruch 1, dadurch gekennzeichnet, daß n gleich 1 ist, R unter einem Cycloalkyl- und einem Cycloalkenylrest mit 6 bis 8 Kohlenstoffatomen ausgewählt ist und A den Rest $—CH_2—$ oder $—(CH_2)_2$ bedeutet.

4. Pharmazeutische Formulierung nach Anspruch 1, dadurch gekennzeichnet, daß das Imidazol unter 1-Cyclohexylethylimidazol und 1-Cyclohex-3-enylmethylimidazol und deren pharmazeutisch unbedenklichen Säureadditionssalzen ausgewählt ist.

5. Pharmazeutische Formulierung nach einem der Anspruch 1 bis 4, dadurch gekennzeichnet, daß sie sich in der Form einer Tablette, Kapsel oder einer parenteral verträglichen injizierbaren Lösung oder Suspension befindet.

6. Pharmazeutische Formulierung nach Anspruch 1, enthaltend 1-Cyclohexylmethylimidazol oder ein pharmazeutisch verträgliches Säureadditionssalz davon, zusammen mit einem pharmazeutisch unbedenklichen Träger dafür.

7. Pharmazeutische Formulierung nach Anspruch 1, enthaltend 1-Cyclohexylmethylimidazol oder ein pharmazeutisch verträgliches Säureadditionssalz davon in Form einer Tablette, Kapsel oder parenteral verträgliche injizierbaren Lösung oder Suspension.

8. Pharmazeutische Formulierung nach Anspruch 1, enthaltend 1-Cyclooctylmethylimidazol oder ein pharmazeutisch verträgliches Säureadditionssalz davon, zusammen mit einem pharmazeutisch unbedenklichen Träger dafür.

9. Pharmazeutische Formulierung nach Anspruch 1, enthaltend 1-Cyclooctylmethylimidazol oder ein pharmazeutisch verträgliches Salz davon in Form einer Tablette, Kapsel oder parenteral verträglichen injizierbaren Lösung oder Suspension.

10. Tablette nach Anspruch 9, dadurch gekennzeichnet, daß sie 50 bis 500 mg des Imidazols oder eines pharmazeutisch verträglichen Salzes davon enthält, wobei die Menge die Menge der vorliegenden Imidazolbase ist.

11. Verfahren zur Herstellung von Formulierungen nach Anspruch 1, dadurch gekennzeichnet, daß man ein Imidazol oder ein pharmazeutisch verträgliches Säureadditionssalz gemäß der Definition in Anspruch 1 mit einem pharmazeutisch verträglichen Träger dafür vermischt.

12. Imidazol, gekennzeichnet, durch die Formel

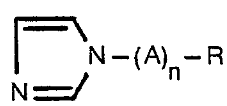

worin n gleich 0 ist und R einen Cycloalkyl- oder Cycloalkenylrest mit 4 bis 9 Kohlenstoffatomen darstellt und gegebenenfalls durch einen oder mehrere Alkylreste mit jeweils 1 bis 4 Kohlenstoffatomen substituiert ist, mit der Maßgabe, daß R nicht unsubstituiertes Cyclohexyl ist, oder ein pharmazeutisch unbedenkliches Säureadditionssalz davon.

13. Imidazol nach Anspruch 12 mit der weiteren Maßgabe, daß R nicht substituiertes Cyclohexyl ist.

14. Imidazol, gekennzeichnet durch die Formel

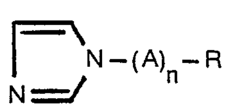

worin n für 1 steht und A und R zusammen einen Alkenylrest mit 5 bis 9 Kohlenstoffatomen bilden, oder ein pharmazeutisch unbedenkliches Säureadditionssalz davon.

15. Imidazol nach Anspruch 12, welches 1-Cyclopentylimidazol oder 1-Cycloheptylimidazol ist, oder ein pharmazeutisch unbedenkliches Säureadditionssalz davon.

16. Imidazol nach Anspruch 14, welches 1-(Pent-2-enyl)-imidazol oder 1-(Pent-4-enyl)imidazol ist, oder ein pharmazeutisch unbedenkliches Säureadditionssalz davon.

17. Pharmazeutische Formulierung, welche eine Verbindung nach einem der Ansprüche 12 bis 16 enthält, und einen pharmazeutisch unbedenklichen Träger dafür.

18. Imidazol nach Formel I gemäß der Definition in Anspruch 1, wobei das Imidazol die freie Base oder ein pharmazeutisch unbedenkliches Salz davon ist, zur Verwendung in der Behandlung oder Prophylaxe von thrombo-embolischen Erkrankungen eines Säugetiers oder Säugetiergewebes.

19. Imidazol nach Anspruch 18, dadurch gekennzeichnet, daß n für 1 steht, A einen geraden oder verzweigten, gesättigten oder ungesättigten acyclischen Kohlenwasserstoffrest mit 1 bis 3 Kohlenstoffatomen bedeutet und R einen Cycloalkyl- oder Cycloalkenylrest mit 4 bis 9 Kohlenstoffatomen darstellt, der gegebenenfalls durch einen oder mehrere Alkylreste mit jeweils 1 bis 4 Kohlenstoffatomen substituiert ist.

20. Imidazol nach Anspruch 18, worin $(A)_n$ und R zusammen einen Alkenylrest mit 5 bis 9 Kohlenstoffatomen bilden.

21. Imidazol nach Anspruch 18, worin n für 0 steht.

22. 1-Cyclooctylmethylimidazol oder ein pharmazeutisch verträgliches Säureadditionssalz davon zur Verwendung in der Behandlung oder Prophylaxe von thrombo-embolischen Erkrankungen bei einem Säugetier.

23. 1-Cyclooctylmethylimidazol oder ein pharmazeutisch verträgliches Säureadditionssalz davon zur Verwendung in der Behandlung oder Prophylaxe von Thromben bei einem Säugetier.

24. 1-Cyclooctylmethylimidazol oder ein pharmazeutisch verträgliches Säureadditionssalz davon zur Verwendung in der Behandlung oder Prophylaxe eines Myocardialinfarkts bei einem Säugetier.

25. 1-Cyclohexylmethylimidazol oder ein pharmazeutisch verträgliches Säureadditionssalz davon zur Verwendung in der Behandlung oder Prophylaxe von thrombo-embolischen Erkrankungen bei einem Säugetier.

26. Pharmazeutische Formulierung nach einem der Ansprüche 1 bis 10 zur Verwendung in der Behandlung oder Prophylaxe einer thrombo-embolischen Erkrankung bei einem Säugetier.